# EUROPEAN PATENT SPECIFICATION

(11) **EP 0 675 764 B1**
(45) Date of publication and mention of the grant of the patent: **23.04.2003**
(21) Application number: 94902940.9
(22) Date of filing: 22.12.1993
(51) Int. Cl.: B05B 5/043, A61M 15/00

(54) **DISPENSING DEVICE**
AUSGABEVORRICHTUNG
DISTRIBUTEUR

(30) Priority: 22.12.1992 GB 9226717
(43) Date of publication of application: 11.10.1995
(73) Proprietor: BATTELLE MEMORIAL INSTITUTE, Columbus Ohio 43201-2693 (US)
(72) Inventor: COFFEE, Ronald Alan, Haslemere, Surrey GU27 1HA (GB)
(74) Representative: Clark, Jane Anne
(86) International application number: GB9302634
(87) International publication number: WO94014543

(56) References cited:
- WO-A-91/07232
- DE-A- 2 008 769
- US-A- 4 044 404
- US-A- 4 266 721
- TRANSACTIONS OF THE AMERICAN SOCIETY OF AGRICULTURAL ENGINEERS, vol.9, no.4, 1966, SAINT JOSEPH, MICH US pages 501 - 506 S. E. LAW AND H. D. BOWEN 'charging liquid spray by electrostatic induction'

## Description

The invention relates to a dispensing device for comminuting a liquid and the uses of such a device, especially in medicine.

Dispensing devices are known which produce a finely divided spray of liquid droplets by electrostatic (more properly referred to as 'electrohydrodynamic') means. The droplet spray in such devices is generated by the application of an electric field to a liquid at a spray head or spray edge. The potential of the applied electric field is sufficiently high to provide comminution of the liquid from the spray head. The droplets produced are electrically charged and thus are prevented from coagulating by mutual repulsion.

Electrohydrodynamic sprayers have potential uses in many areas, including agriculture and the automotive industry and also for dispensing cosmetics and medicines.

United Kingdom patent number 1569707 describes such an electrohydrodynamic spray device principally for use in crop spraying.

United Kingdom patent number 2018627B discloses an electrohydrodynamic spray device wherein the charged droplet spray is fully or partially electrically discharged by means of an earthed electrode having a sharp or pointed edge and located downstream of the spray head. European Patent number 0234842 also uses this technology and relates to an inhaler in which charged droplet spray is discharged prior to inhalation by means of a sharp or pointed discharge electrode carrying an opposite charge to the droplet spray and located downstream of the spray head. The droplets are discharged to facilitate droplet deposition into the respiratory tract by preventing deposition of charged droplets onto the mouth and throat of the user.

A common feature of all known electrohydrodynamic spray devices is that the electric charge used to generate the spray is applied directly to the spray head. It has now surprisingly been found that the direct application of the field is not essential and that the electrohydrodynamic comminution of a liquid may be accomplished by inducing the required electric charge at the spray head. In addition and advantageously, it has been found that the comminutions produced can be partially or fully discharged prior to use.

This method of induced charging has been found to provide better comminution of liquids having lower electrical resistivity.

According to one aspect of the invention as defined in claim 1, there is provided a dispensing device comprising an electrohydrodynamic comminution site means for supplying liquid to the comminution site and means spaced from the comminution site for causing comminution of the liquid by charging the comminution site to an electrical potential by inducing electrical charge at the comminution site, characterised by further comprising means for partially or fully electrically discharging comminuted matter to inhibit impact on the induction charging means.

In another aspect as defined in claim 17 there is provided a method of dispensing comminuted matter which comprises: supplying liquid to a comminution site and causing comminution of the liquid by charging the comminution site to an electrical potential by induction using charging means spaced from the comminution site, characterised by partially or fully electrically discharging comminuted matter to inhibit impact on the induction charging means.

US.A. 4 044 404 represents the prior art as referred to in the preamble of claim 1 and claim 17.

The comminution site may be any conventional electrohydrodynamic comminution site such as a surface or edge generally provided by a thin capillary tube, a nozzle or a slot defined by two parallel plates.

Appropriate means for supplying liquid to the comminution site include mechanical or electrically powered pumps which are capable of providing the required flow rate of liquid to the comminution site such as a syringe pump or the electrically powered pump described in EP 0029301.

The comminution means of a device embodying the invention can be used with a large range of flow rates, but generally operates with flow rates in the range of between 0.1 to 500µL per second, such as 0.5 to 5µL per second, especially for inhaled administration, or 10 to 200µL per second, especially for agricultural use.

The means for inducing the electrical charge at the comminution site may be any conventional source of electrical charge which in use is capable of inducing a charge sufficient to comminute the liquid from the comminution means, such as a high voltage generator or a piezo-electric generator. The voltage charge usually required is of the order of 1-20 kilovolts, for example 10 kilovolts.

One suitable means for partially or fully electrically discharging comminuted matter is a sharp or pointed discharge electrode located downstream of the comminuted matter.

The sharp or pointed discharge electrode may be earthed or it may be maintained at a polarity opposite to that of the induction charging means by connection to a suitable charging means. In either case, in use, the comminution is partially or fully discharged by a cloud of charged ions produced from the surrounding air and having an opposite electrical charge to the comminuted spray. The ion cloud is attracted towards, collides with and thereby partially or fully discharges, the spray.

In one particularly advantageous embodiment of the invention, the means for fully or partially discharging the comminution is provided by a combination of the sharp or pointed discharge electrode and at least one capacitor, the capacitor acting to absorb the charge from the gaseous ions from the sharp or pointed discharge electrode until the induced comminution of the liquid is established and to absorb the ions until it reaches a predetermined potential at which the capacitor ceases to absorb the ions thereby allowing them to partially or fully discharge the comminution.

Generally, the capacitor is chosen to have a time constant having the same order as the time required to establish the comminution spray cloud. Thus the time constant will have a value, in seconds, which is the product of the capacitance, C and the resistance, R, of the capacitor.

The value of CxR for the capacitor is chosen so that the capacitor will charge until it reaches a prearranged potential sufficient to modify the electric field, the capacitor then discharges towards the established spray cloud. Generally, the time constant required will be of the order of seconds or a number of milliseconds. For example, a capacitor of 0.1 microfarad with a resistance of 10 megohms will produce a time constant of one second.

In another embodiment, the induction charging means comprises an electrode and means for providing an electrical potential at the electrode, the electrode having a first surface for producing, in response to the electrical potential applied in use by the electrical potential applying means, an electrical field for inducing the electrical potential for causing comminution of liquid at the comminution site and for causing comminuted matter to substantially by-pass the first surface, and the electrical discharging means comprising a second surface of the electrode for producing, in response to the electrical potential applied in use by the electrical potential applying means, an ionic electric discharge to fully or partially discharge the comminuted matter.

The electrode may be an annular electrode located coaxially with respect to the path of comminuted matter from the comminution site, a surface of the annular electrode closest to the comminution site forming said first surface and a surface of the annular electrode remote from the comminution site being shaped so as to form said second surface.

In operation the electric field pattern provided by the upper surface of the annular electrode is such that the comminution is directed onto an axial flight path with respect to the annular electrode and is provided with sufficient inertial force to substantially bypass the first surface, the comminution is then fully or partially discharged by the gaseous ions produced by the second surface.

A device embodying the invention may be used to dispense liquids comprising components useful for human or animal health care, such as medicaments for pharmaceutical or public health care use or medically useful compounds such as anaesthetics.

Suitable liquids include liquids comprising components for agricultural use such as pesticides or biocides.

Suitable liquids include liquid cosmetic formulations.

Other suitable liquids include paints and inks. Also included are liquids for providing aromas.

Preferred liquids are pharmaceutically active liquids.

In an embodiment the comminution means of the dispenser may provide in operation droplets within the range of from about 0.1 to about 500 microns in diameter: More usually from 0.1 to 200 microns, such as 1.0 to 200 microns: Examples include droplets within the range of 5.0 to 100, 0.1 to 25, 0.5 to 10 or 10 to 20 microns. A favoured range for inhaled administration is 0.1 to 25 or 0.5 to 10 microns, especially for administration to the lower respiratory tract, and 10 to 25 microns, especially for administration to the upper respiratory tract.

For a given liquid the diameter of the droplets can be controlled by varying the applied voltage and liquid flow rate using routine experimental procedures.

Liquids having viscosities within the range of from 1 to 500 centipoise and resistivities in the range of from 10² - 10⁸ ohm metres can be comminuted by the present device.

As stated above, charging the comminution site by induction has been found to provide better comminution of liquid having a lower electrical resistivity, such as is the case of aqueous solvents, including solvent mixtures, and solutions thereof and low resistivity organic solvents such as alcohols.

One favoured use of a device embodying the invention is for the dispensation of a comminuted liquid for inhalation.

A device embodying the invention may be adapted into any form which dispenses comminuted liquid for inhalation, for both medicinal and non-medicinal use.

Non-medicinal inhalation uses includes dispensing perfumes and aromas.

Preferably, for the inhaled delivery of a medicament the device is in the form of an inhaler. A preferred liquid for such a device is therefore a liquid medicament formulation adapted for inhaled administration.

Medicaments suitable for adaption for inhaled administration include those used for the treatment of disorders of the respiratory tract, such as reversible airways obstruction and asthma and those used in the treatment and/or prophylaxis of disorders associated with pulmonary hypertension and of disorders associated with right heart failure by inhaled delivery.

One problem associated with inhalers is coordinating the release of the comminuted spray with inhalation by the user. An embodiment of the present invention provides a flap valve and the discharge electrode projects from the plane of the flap valve, the flap valve being pivotally mounted so as to be movable between closed and open positions in response to inhalation by a user such that, when the flap valve pivots to the open position, the discharge electrode is pivoted into the path of droplets of comminuted matter, facilitating coordination of the release of the spray with inhalation.

The discharging means may comprise an electrical discharge electrode, means for providing an electrical discharge potential at the electrical discharge electrode and at least one capacitor for absorbing charge from gaseous ions generated by the electrical discharge electrode until a comminution has been established by the comminution site and for ceasing to absorb gaseous ions after establishment of a liquid comminution to allow partial or full discharge of the comminution by gaseous ions.

In an embodiment, a valve means located so as to open and close the conduit, suitably within the conduit is arranged to be opened by inhalation of the user and to then activate the discharging means.

The discharging means may comprise a discharge electrode coupled to said valve means such that when said valve means is opened in response to inhalation by a user, the discharge electrode is exposed to partially or fully electrically discharge droplets formed by the comminution site.

When the discharging means is a sharp edged or pointed electrode, the discharging means is preferably operationally attached to the valve means such that when the value means opens the discharge electrode is thereby exposed to the comminution. A suitable valve means is a flap valve.

In an embodiment the sharp edged or pointed electrode is fixed so as to extend upwards from the plane of the flap valve, the flap valve being pivotally fixed so as to open and close the conduit, such that when the flap valve pivots open the discharge electrode pivots into the flight path of the comminuted liquid.

In an embodiment the invention provides an inhaler, the inhaler comprising an electrohydrodynamic comminution site, a means for supplying liquid to the comminution site, a means for charging the comminution site, a sharp edged or pointed electrode for partially or fully discharging the liquid comminution and a conduit through which the liquid comminution is administered, the conduit having a valve means activated by inhalation of the user, wherein the valve means comprises a flap shaped to seal the conduit, the flap being pivotally mounted so as to open and close the conduit, the sharp edged or pointed electrode extending upwards from the plane of the flap valve, such that in use the flap valve pivots open and the discharging means pivots into the flight path of the comminuted liquid upon inhalation by the user.

When a device embodying the invention comprises a sharp edged or pointed electrode, the arrangement suitably provides that the sharp edged or pointed electrode is electrically shielded from the comminution when the valve means is closed. One particular method of achieving this is that the sharp edged or pointed electrode pivots into a recess formed in the charging means when the valve means closes.

When used herein 'a comminution' includes a liquid droplet spray.

When used herein 'medicament' includes proprietary medicines, pharmaceutical medicines and veterinary medicines.

When used herein, unless more specifically defined herein, 'inhaled administration' includes administration to and via the upper respiratory tract, including the nasal mucosa, and the lower respiratory tract.

The description 'sharp edged or pointed' when used herein in relation to operational parts of the device, such as a discharge electrode, also includes electrical equivalents thereof and hence includes shapes such as ridges and the like, the essential requirement being is that in the embodiment the operational part of the device has, or a component or feature of the device has, dimensions which will give rise to a sufficiently high electrical field strength so as to exceed the breakdown strength of the air. This topic is theoretically described in "Depositional Control of Macroscopic Particles by High Strength Electric Field Propulsion" by R A Coffee, in "Transactions of the Institution of Electrical and Electronic Engineers, Industry Applications, USA", Vol. IA-10 pp 511 to 519, July/August 1974. An example is an electrical field strength of approximately 3 million volts per meter.

Liquid medicinal formulations for use in a device embodying the invention may be formulated according to conventional procedures, such as those disclosed in the US Pharmacopoeia, the European Pharmacopoeia, 2nd Edition, Martindale The Extra Pharmacopoeia, 29th Edition, Pharmaceutical Press and the Veterinary Pharmacopoeia.

Liquid cosmetic formulations for use in a device embodying the invention may be formulated according to conventional procedures, such as those disclosed in Harry's cosmeticology, 9th Edition, 1982, George Goodwin, London.

Embodiments of the present invention will now be described, by way of example, with reference to the accompanying drawings in which:

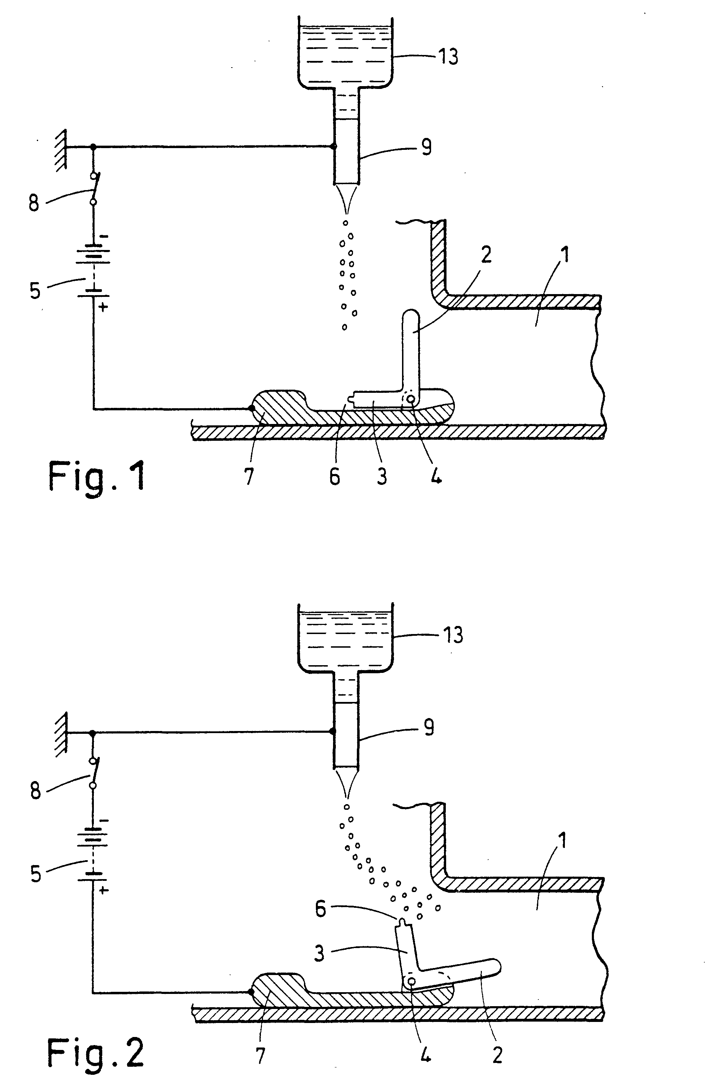

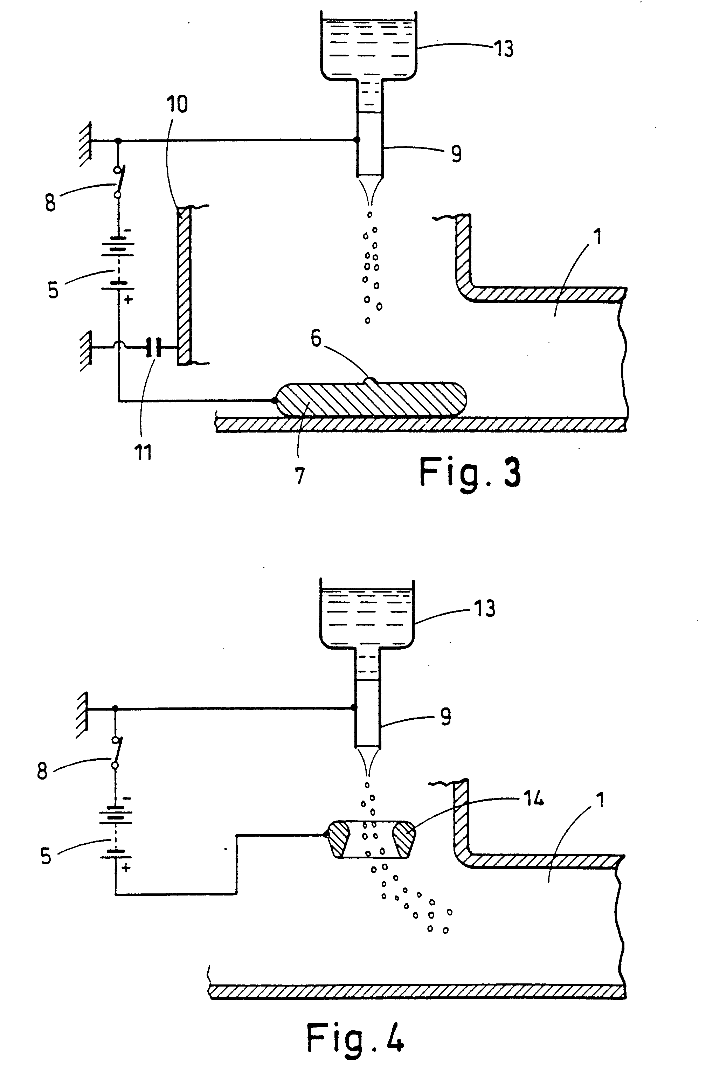

Figures 1 and 2 show a first embodiment of a device in accordance with the invention and Figures 3 to 5 show further embodiments of a device in accordance with the invention.

In Figures 1 and 2, a device embodying the invention is illustrated in which a pressure reduction created by the action of breathing through a suitable ducting (1) causes a lightweight flap (2), balanced by a second member (3) pivoted at a hinge (4) and connected to a dc high voltage supply of either polarity (5) to revolve through a sufficient degree of arc to allow the second member of the flap to become exposed to the electric field and then create gaseous ions.

The flap valve thus has two actions: (a) it opens an air passage (1) to facilitate a flow of droplets; and (b) it simultaneously rotates a balancing member (3) attached to the flap (2) through a sufficient degree of arc to expose a ridge, or nipple (6) having one dimension of less than about 1.0 mm radius of curvature.

The ridge, or nipple (6) may be made of any conducting, or semi-conducting material such as metal, or carbon-loaded plastic, and is connected to a source of high voltage (5). When not actuated by breathing, the ridge will be electrically screened by the surface of a flat electrode (7), also connected to the high voltage source (5). In this position the electrode (7) may be switched on or off by a simple switch (8).

When switched on, the electrode induces a potential of opposing polarity at the tip of a nearby nozzle (9). This induced potential causes liquid at the tip of the nozzle to emerge as a fast jet which breaks up into charged droplets. The nozzle (9) is connected to earth.

In this device (a) the flat valve (2) allows droplets to be inhaled only when the valve is actuated by the act of breathing; (b) the principle of induction, rather than direct, nozzle charging improves the control of droplet size and maximum flow rate, for those liquids would otherwise be difficult to atomize electrohydrodynamically; and (c) avoids the opposite polarity droplets otherwise being so strongly attracted to the source of the induced voltage (7) that the droplets would not be available for delivery by inhalation, or other forms of deposition onto target surfaces.

Figure 3 shows another device embodying the invention in which one or more electrically floating conducting or semi-conducting surfaces (10), attached to one or more capacitors (11) are used to attract and capture gaseous ions so that the electric field created by the electrode (6) acts directly upon the nozzle (8) without impingement of gas ions. Such gas ions, if allowed to reach the nozzle unimpeded would be expected to modify the electric field surrounding the nozzle so as to prevent the emerging liquid from forming the necessary jet of liquid for atomization by the electrohydrodynamic method. The capacitor(s) is chosen to have a time constant of the same order as the time required to establish a spray cloud. This time constant will have a value, in seconds, which is the product of the capacitance, C and the resistance, R, of the capacitor. The value of CxR is thus chosen so that the capacitor will charge by bombardment of gaseous ions, until it reaches a sufficient potential to modify the electric field and to re-direct the ions toward the established spray cloud. Generally, the time constant required will be of the order of seconds or a number of milliseconds. For example, a capacitor of 0.1 microfarad with a resistance of 10 megohms will produce a time constant of one second. Figure 3 shows one configuration that will create the required induction potential at the nozzle when the electrode (7) is energized and, after a suitable period, dependent upon the position and time constant of the capacitor(s) will then re-arrange the field to allow gaseous ions to migrate into the spray cloud so as to modify the charges on all droplets to a lower (optimal) or approximately zero value. Such droplets may then be readily inhaled.

The charged droplets are prevented from impinging upon the high voltage electrode (7) by the action of fast moving gaseous ions. These ions are created by the combination of electrode voltage, say one to ten kilovolts dc, and the radius of curvature of the small dimension of the ridge or nipple (6) on the balancing member (3) and by juxtaposition of the nozzle (9), the electrode (7) and the capacitor(s) (11) which latter may be used to increase the degree of control of the shape of the field and the timing of the essential reshaping process.

Liquid is supplied to the nozzle (9) from either a container (13) by gravity feed, or by mechanical pumping, or by an electrokinetic pumping device.

The liquid is supplied to the nozzle and the induced voltage applied by the electrode (7) before the electric field is modified to create gaseous ions by the actuation of the flap-valve (2) and/or the capacitor(s) (11). Then, at any time after the spray cloud is developed, the breath-actuated valve and/or the capacitor(s) is actuated, whereupon the droplet trajectories are modified; moving away from their direct flight to the electrode (7), through the required angle, say to flow by viscous drag in the air movement caused by normal breathing. This action is virtually instantaneous due to the extremely low inertial forces on droplets used for inhalation therapy, which are generally less than about 10.0 µm in diameter for drug inhalation.

An alternative method of creating the required induction potential to atomize the liquid and subsequently discharge the droplets before impingement upon the induction electrode is to use an induction electrode (14) such as, for example, a ring with two distinct cross-sectional radii of curvature, as shown in Figure 4. This method may be used with or without a flap valve (2), or field modifying capacitor(s) (11). The larger radius faces toward the nozzle tip, whilst the smaller radius (say less than about 1.0mm) faces away from the nozzle (9). It has been found that, by very careful design of the field pattern, charged droplets may have sufficient inertial force to pass through a gap in the electrode (14) without immediate impingement. Although these droplets are then almost immediately forced back to impinge upon the electrode, they may be prevented from doing so by the neutralizing action of the fast moving gaseous ions. It has been further discovered that production of gaseous ions by gas breakdown at the smaller radius of curvature may be delayed by maintaining the field strength at the electrode below the critical value until the charged droplets enter the field, whereupon they will increase the field strength to the critical value and immediately trigger the droplet discharge process.

The critical field strength and shape is a function of: electrode position, shape, and voltage; the relative positions and potentials of the nozzle and capacitor(s) surfaces and the degree and position of space charge potential created by the charged droplets.

It has also been found that the methods of controlled field modification (with time) disclosed herein may be so set as to both discharge and, if required, to recharge the droplets to an optimal value. This could be of importance in, say, ensuring accurate deposition of droplets within a human lung, where both the droplet's mass, and its charge have controlling influence upon the zones of deposition within the system of airways through which the droplets pass during inhalation.

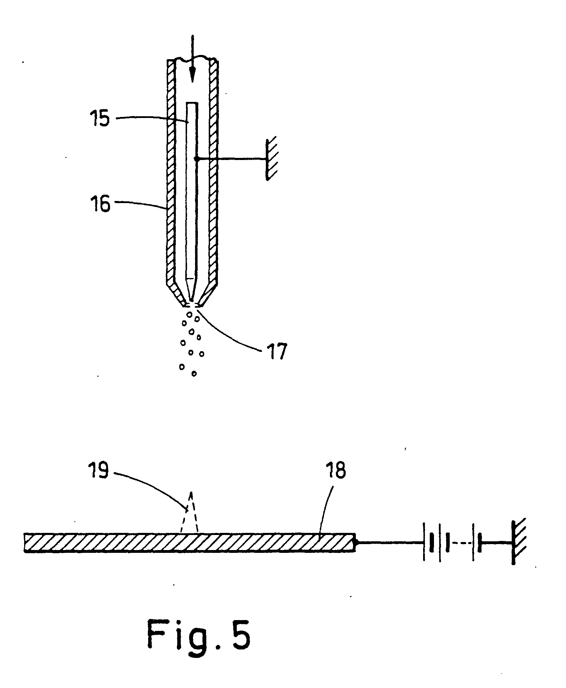

A particular example of the device and its operation is shown in Figure 5: An earthed needle, (15) concentrically located within a non-conducting sleeve (16) allowed liquid to flow (by gravity or other light pressure) to an outlet nozzle (17) where the liquid was exposed to a strong convergent electric field provided by a high potential supplied to the flat, smooth surface of electrode (18). This resulted in an induced electrohydrodynamic (EHD) communition of the liquid emerging from capillary nozzle (17).

After the communition was established ( and within less than one second) a sharp element (19) of the induction electrode (18) was exposed.

The exposure of (19) above the smooth surface of (18) produced gaseous ions of the polarity of the high voltage dc. generator (20). Since the EHD spray cloud was induced from an earthed electrode-nozzle (17), the gaseous ions and the spray droplets have opposing polarities. And as the gaseous ions have much greater mobility in the electric field containing both droplets and ions, the droplets were bombarded and hence electrically discharged.

In the experiment described, the distance between tip of nozzle (17) and flat electrode was 30mm. When the sharp electrode (19) was positioned to discharge the droplets, the distance between tip of nozzle (17)and needle-tip (19) was 23mm. The liquid flow-rate was 1.34 µl/sec. The high voltage source was set at a negative potential of 10.7 kilovolts.

The liquid used was 80% ethanol and 20% polyethylene glycol (200), having a viscosity of 2.2c Poise, a surface tension of 25.0m N/m, a resistivity of 1.7 x 10³ohm.m and a density of 0.86 kg/litre.

The discharging effect was assessed to be essentially 100 per cent.

## Claims

1. A dispensing device comprising an electrohydrodynamic comminution site (9; 15, 17), means (13; 16) for supplying liquid to the comminution site and means (5, 2; 5, 14; 18) spaced from the comminution site for causing comminution of the liquid by charging the comminution site to an electrical potential by inducing electrical charge at the comminution site, **characterised by** further comprising means (5, 6; 5, 14; 19) for partially or fully electrically discharging comminuted matter to inhibit impact on the induction charging means.

2. A device according to claim 1, wherein the induction charging means comprises a high voltage or piezoelectric generator (5).

3. A device according to claim 1 or 2, wherein the partial or full electrical discharging means comprises an electrical discharge electrode (6, 19) located downstream of the comminution site.

4. A device according to claim 3, comprising means for maintaining the electrical discharge electrode at ground potential or at a polarity opposite to that to which the induction charging means is arranged to charge the comminution site.

5. A device according to claim 1 or 2, wherein the induction charging means comprises an electrode (114) and means (5) for providing an electrical potential at the electrode, the electrode having a first surface for producing, in response to the electrical potential applied in use by the electrical potential applying means, an electrical field for inducing the electrical potential for causing comminution of liquid at the comminution site (9) and for causing comminuted matter to substantially by-pass the first surface, and the electrical discharging means comprises a second surface of the electrode for producing, in response to the electrical potential applied in use by the electrical potential applying means, an ionic electric discharge to fully or partially discharge the comminuted matter.

6. A device according to claim 5, wherein the electrode is an annular electrode (14) located coaxially with respect to the path of comminuted matter from the comminution site (9), a surface of the annular electrode closest to the comminution site (9) forming said first surface and a surface of the annular electrode remote from the comminution site being shaped so as to form said second surface.

7. A device according to claim 1 or 2, wherein the means for fully or partially electrically discharging droplets prior to any comminuted matter impacting the induction charging means comprises an electrical discharge electrode (6), means (5) for providing an electrical discharge potential at the electrical discharge electrode and at least one capacitor (11) for absorbing charge from gaseous ions generated by the electrical discharge electrode until a comminution has been established by the comminution site (9) and for ceasing to absorb gaseous ions after establishment of the comminution to allow partial or full discharge of the comminution by gaseous ions.

8. An inhaler comprising a device according to claim 1 or 2, wherein said means for partially or fully electrically discharging comminuted matter is operable to partially or fully discharge comminuted matter in response to inhalation by a user and a conduit is provided for supplying fully or partially electrically discharged comminuted matter to the user.

9. An inhaler according to claim 8, further comprising valve means (2) for closing the conduit, the valve means being arranged to be opened by inhalation by a user and so as to activate said electrical discharging means (5, 6) upon opening.

10. An inhaler according to claim 9, wherein said electrical discharge means (5, 6) for partially or fully discharging comminuted matter comprises a discharge electrode (6) coupled to said valve means (2) such that when said valve means is opened in response to inhalation by a user, the discharge electrode is exposed to partially or fully electrically discharge comminuted matter formed by the comminution site (9).

11. An inhaler according to claim 10, wherein the valve means is a flap valve (2) and the discharge electrode (6) projects from the plane of the flap valve, the flap valve being pivotally mounted (4) so as to be movable between closed and open positions in response to inhalation by a user such that, when the flap valve pivots to the open position, the discharge electrode (6) is pivoted into the path of the comminuted matter.

12. An inhaler according to claim 10 or 11, wherein the discharge electrode (6) is arranged so as to move into a recess formed in said charging means when said valve means moves to its closed condition.

13. An inhaler comprising a device in accordance with any one of claims 1 to 7 for dispensing comminuted matter for inhalation.

14. An inhaler according to any one of claims 8 to 13 adapted to dispense comminuted matter for delivery to the upper respiratory tract.

15. An inhaler according to claim 14 adapted to deliver droplets having a diameter from about 10 to about 25 micrometres to the upper respiratory tract.

16. A device according to any one of claims 1 to 7 adapted to dispense perfumes, aromas or to enable inhaled delivery of a medicament.

17. A method of dispensing comminuted matter which comprises: supplying liquid to a comminution site and causing comminution of the liquid by charging the comminution site to an electrical potential by induction using charging means spaced from the comminution site, **characterised by** partially or fully electrically discharging comminuted matter to inhibit impact on the induction charging means.

18. A method according to claim 17 which comprises supplying the liquid to be comminuted and inducing the electrical potential at the comminution site so as to provide droplets for delivery to the upper respiratory tract.

19. A method according to claim 17 which comprises supplying the liquid to be comminuted and inducing the electrical potential at the comminution site so as to provide droplets having a diameter in the range of from about 10 to about 25 micrometres for delivery to the upper respiratory tract.

## Patentansprüche

1. Abgabevorrichtung, die eine elektrohydrodynamische Zerstäubungsstelle (9; 15, 17), eine Einrichtung (13; 16) zur Lieferung einer Flüssigkeit an die Zerstäubungsstelle und eine Einrichtung (5, 2; 5, 14; 18) aufweist, die von der Zerstäubungsstelle beabstandet ist, zum Bewirken einer Zerstäubung der Flüssigkeit durch Aufladen der Zerstäubungsstelle auf ein elektrisches Potential, indem eine elektrische Ladung an der Zerstäubungsstelle induziert wird, **dadurch gekennzeichnet, daß** sie ferner eine Einrichtung (5, 6; 5, 14; 19) zur teilweisen oder vollständigen elektrischen Entladung der zerstäubten Substanz aufweist, um ein Auftreffen auf die Induktionsladeeinrichtung zu verhindern.

2. Vorrichtung nach Anspruch 1, wobei die Induktionsladeeinrichtung einen Hochspannungs- oder piezoelektrischen Generator (5) aufweist.

3. Vorrichtung nach Anspruch 1 oder 2, wobei die Einrichtung zur teilweisen oder vollständigen elektrischen Entladung eine elektrische Entladungselektrode (6, 19) aufweist, die stromabwärts von der Zerstäubungsstelle angeordnet ist.

4. Vorrichtung nach Anspruch 3, die eine Einrichtung aufweist, die die elektrische Entladungselektrode auf Massepotential oder auf einer Polarität hält, die entgegengesetzt zu jener ist, für die die Induktionsladeeinrichtung eingerichtet ist, um die Zerstäubungsstelle zu laden.

5. Vorrichtung nach Anspruch 1 oder 2, wobei die Induktionsladeeinrichtung eine Elektrode (114) und eine Einrichtung (5) zur Bereitstellung eines elektrischen Potentials an der Elektrode aufweist, wobei die Elektrode eine erste Oberfläche aufweist, um als Reaktion auf das elektrische Potential, das im Gebrauch durch die Einrichtung zum Anlegen eines elektrischen Potentials angelegt wird, ein elektrisches Feld zu erzeugen, zur Induktion des elektrischen Potentials zum Bewirken einer Flüssigkeitszerstäubung an der Zerstäubungsstelle (9) und zum Bewirken, daß eine zerstäubte Substanz die erste Oberfläche im wesentlichen vermeidet, und die elektrische Entladungseinrichtung eine zweite Oberfläche der Elektrode aufweist, um als Reaktion auf das elektrische Potential, das im Gebrauch durch die Einrichtung zum Anlegen eines elektrischen Potentials angelegt wird, eine ionische elektrische Entladung zur teilweisen oder vollständigen Entladung der zerstäubten Substanz zu erzeugen.

6. Vorrichtung nach Anspruch 5, wobei die Elektrode eine ringförmige Elektrode (14) ist, die bezüglich des Weges der zerstäubten Substanz von der Zerstäubungsstelle (9) koaxial angeordnet ist, wobei eine Oberfläche der ringförmigen Elektrode, die der Zerstäubungsstelle (9) am nächsten ist, die erste Oberfläche bildet, und eine Oberfläche der ringförmigen Elektrode, die von der Zerstäubungsstelle entfernt ist, so geformt ist, daß sie die zweite Oberfläche bildet.

7. Vorrichtung nach Anspruch 1 oder 2, wobei die Einrichtung zur teilweisen oder vollständigen elektrischen Entladung von Tröpfchen, bevor irgendeine zerstäubte Substanz auf die Induktionsladeeinrichtung auftrifft, eine elektrische Entladungselektrode (6), eine Einrichtung (5) zur Bereitstellung eines elektrischen Entladungspotentials an der elektrischen Entladungselektrode und mindestens einen Kondensator (11) zur Absorption einer Ladung von Gasionen, die durch die elektrische Entladungselektrode erzeugt werden, bis eine Zerstäubung durch die zerstäubungsstelle (9) hergestellt worden ist, und zur Einstellung der Absorption von Gasionen nach dem Aufbau der Zerstäubung aufweist, um eine teilweise oder vollständige Entladung der Zerstäubung durch Gasionen zuzulassen.

8. Inhalationsapparat, der eine Vorrichtung nach Anspruch 1 oder 2 aufweist, wobei die Einrichtung zur teilweisen oder vollständigen elektrischen Entladung einer zerstäubten Substanz betriebsfähig ist, die zerstäubte Substanz als Reaktion auf eine Inhalation durch einen Benutzer teilweise oder vollständig zu entladen, und ein Kanal zur Lieferung der teilweise oder vollständig elektrisch entladenen zerstäubten Substanz an den Benutzer vorgesehen ist.

9. Inhalationsapparat nach Anspruch 8, der ferner eine Ventileinrichtung (2) zum Schließen des Kanals aufweist, wobei die Ventileinrichtung eingerichtet ist, durch eine Inhalation durch einen Benutzer geöffnet zu werden und damit die elektrische Entladungseinrichtung (5, 6) beim Öffnen zu aktivieren.

10. Inhalationsapparat nach Anspruch 9, wobei die elektrische Entladungseinrichtung (5, 6) zur teilweisen oder vollständigen Entladung der zerstäubten Substanz eine Entladungselektrode (6) aufweist, die mit der Ventileinrichtung (2) so gekoppelt ist, daß wenn die Ventileinrichtung als Reaktion auf eine Inhalation durch einen Benutzer geöffnet wird, die Entladungselektrode zur teilweisen oder vollständigen elektrischen Entladung der zerstäubten Substanz freigelegt wird, die durch die Zerstäubungsstelle (9) gebildet wird.

11. Inhalationsapparat nach Anspruch 10, wobei die Ventileinrichtung ein Klappenventil (2) ist und die Entladungselektrode (6) aus der Ebene des Klappenventils vorsteht, wobei das Klappenventil schwenkbar angebracht ist (4), so daß es als Reaktion auf eine Inhalation durch einen Benutzer zwischen geschlossenen und offenen Stellungen beweglich ist, so daß wenn das Klappenventil in die offene Stellung schwenkt, die Entladungselektrode (6) in den Weg der zerstäubten Substanz geschwenkt wird.

12. Inhalationsapparat nach Anspruch 10 oder 11, wobei die Entladungselektrode (6) so angeordnet ist, daß sie sich in eine Aussparung bewegt, die in der Ladeeinrichtung ausgebildet ist, wenn sich die Ventileinrichtung in ihren geschlossenen Zustand bewegt.

13. Inhalationsapparat, der eine Vorrichtung nach einem der Ansprüche 1 bis 7 aufweist, zur Abgabe einer zerstäubten Substanz zur Inhalation.

14. Inhalationsapparat nach einem der Ansprüche 8 bis 13, der angepaßt ist, eine zerstäubte Substanz zur Abgabe an die oberen Luftwege abzugeben.

15. Inhalationsapparat nach Anspruch 14, der angepaßt ist, Tröpfchen mit einem Durchmesser von etwa 10 bis etwa 25 Mikrometern an die oberen Luftwege abzugeben.

16. Vorrichtung nach einem der Ansprüche 1 bis 7, die angepaßt ist, Parfüme und Aromen abzugeben oder die Inhalationsabgabe eines Medikaments zu ermöglichen.

17. Verfahren zur Abgabe einer zerstäubten Substanz, das aufweist: Lieferung einer Flüssigkeit an eine Zerstäubungsstelle und Bewirken einer Zerstäubung der Flüssigkeit durch Aufladen der Zerstäubungsstelle auf ein elektrisches Potential durch Induktion, wobei eine Ladeeinrichtung verwendet wird, die von der Zerstäubungsstelle beabstandet ist, **gekennzeichnet durch** eine teilweise oder vollständige elektrische Entladung der zerstäubten Substanz, um ein Auftreffen auf die Induktionsladeeinrichtung zu verhindern.

18. Verfahren nach Anspruch 17, das eine Lieferung der Flüssigkeit, die zerstäubt werden soll, und eine Induktion des elektrischen Potentials an der Zerstäubungsstelle aufweist, um Tröpfchen zur Abgabe an die oberen Luftwege bereitzustellen.

19. Verfahren nach Anspruch 17, das eine Lieferung der Flüssigkeit, die zerstäubt werden soll und eine Induktion des elektrischen Potentials an der Zerstäubungsstelle aufweist, um Tröpfchen mit einem Durchmesser im Bereich von etwa 10 bis etwa 25 Mikrometern zur Abgabe an die oberen Luftwege bereitzustellen.

## Revendications

1. Dispositif distributeur comprenant un site de pulvérisation électrohydrodynamique (9 ; 15, 17), un moyen (13 ; 16) pour amener du liquide au site de pulvérisation et un moyen (5, 2 ; 5, 14 ; 18) espacé du site de pulvérisation pour provoquer la pulvérisation du liquide en chargeant le site de pulvérisation à un potentiel électrique en induisant une charge électrique au site de pulvérisation, **caractérisé par le fait qu'**il comprend en outre un moyen (5, 6 ; 5, 14 ; 19) pour décharger électriquement partiellement ou intégralement la matière pulvérisée pour bloquer l'impact sur le moyen de charge d'induction.

2. Dispositif selon la revendication 1, dans lequel le moyen de charge d'induction comprend une génératrice à haute tension ou piézoélectrique (5).

3. Dispositif selon la revendication 1 ou 2, dans lequel le moyen de décharge électrique partielle ou intégrale comprend une électrode de décharge électrique (6, 19) située en aval du site de pulvérisation.

4. Dispositif selon la revendication 3, comprenant un moyen pour maintenir l'électrode de décharge électrique à un potentiel de terre ou à une polarité opposée à celle à laquelle le moyen de charge d'induction est disposé pour charger le site de pulvérisation.

5. Dispositif selon la revendication 1 ou 2, dans lequel le moyen de charge d'induction comprend une électrode (114) et un moyen (5) pour procurer un potentiel électrique à l'électrode, l'électrode ayant une première surface pour produire, en réponse au potentiel électrique appliqué utilisé par le moyen d'application de potentiel électrique, un champ électrique pour induire le potentiel électrique pour causer la pulvérisation du liquide au site de pulvérisation (9) et pour faire éviter sensiblement à la matière pulvérisée la première surface et le moyen de décharge électrique comprend une seconde surface de l'électrode pour produire, en réponse au potentiel électrique appliqué utilisé par le moyen d'application de potentiel électrique, une décharge électrique ionique pour décharger intégralement ou partiellement la matière pulvérisée.

6. Dispositif selon la revendication 5, dans lequel l'électrode est une électrode annulaire (14) située de manière coaxiale par rapport au trajet de la matière pulvérisée depuis le site de pulvérisation (9), une surface de l'électrode annulaire la plus proche du site de pulvérisation (9) constituant ladite première surface et une surface de l'électrode annulaire éloignée du site de pulvérisation étant formée de façon à constituer ladite seconde surface.

7. Dispositif selon la revendication 1 ou 2, dans lequel le moyen pour décharger électriquement intégralement ou partiellement les gouttelettes avant qu'une matière pulvérisée quelconque ne heurte le moyen de charge d'induction comprend une électrode de décharge électrique (6), un moyen pour procurer un potentiel de décharge électrique à l'électrode de décharge électrique et au moins un condensateur (11) pour absorber la charge d'ions gazeux générée par l'électrode de décharge électrique jusqu'à ce qu'une pulvérisation ait été établie par le site de pulvérisation (9) et pour cesser d'absorber des ions gazeux après établissement de la pulvérisation pour permettre une décharge partielle ou intégrale de la pulvérisation par les ions gazeux.

8. Inhalateur comprenant un dispositif selon la revendication 1 ou 2, dans lequel ledit moyen pour décharger électriquement partiellement ou intégralement la matière pulvérisée est en mesure de décharger partiellement ou intégralement la matière pulvérisée en réponse à l'inhalation par un utilisateur et un conduit est proposé pour fournir de la matière pulvérisée déchargée électriquement intégralement ou partiellement à l'utilisateur.

9. Inhalateur selon la revendication 8, comprenant en outre un moyen de soupape (2) pour fermer le conduit, le moyen de soupape étant agencé pour être ouvert par inhalation par un utilisateur et de façon à activer ledit moyen de décharge électrique (5, 6) lors de l'ouverture.

10. Inhalateur selon la revendication 9, dans lequel ledit moyen de décharge électrique (5, 6) pour décharger partiellement ou intégralement de la matière pulvérisée comprend une électrode de décharge (6) couplée audit moyen de soupape (2) de sorte que lorsque ledit moyen de soupape est ouvert en réponse à une inhalation par un utilisateur, l'électrode de décharge est amenée à décharger électriquement partiellement ou intégralement la matière pulvérisée formée par le site de pulvérisation (9).

11. Inhalateur selon la revendication 10, dans lequel le moyen de soupape est une soupape à clapet (2) et l'électrode de décharge (6) dépasse du plan de la soupape à clapet, la soupape à clapet étant montée de manière pivotale (4) de façon à être mobile entre des positions fermée et ouverte en réponse à l'inhalation par un utilisateur de sorte que, quand la soupape à clapet pivote vers la position ouverte, l'électrode de décharge (6) est pivotée dans la trajectoire de la matière pulvérisée.

12. Inhalateur selon la revendication 10 ou 11, dans lequel l'électrode de décharge (6) est disposée de façon à se déplacer dans un évidemment formé dans ledit moyen de charge lorsque ledit moyen de soupape se déplace vers son état fermé.

13. Inhalateur comprenant un dispositif en conformité avec l'une quelconque des revendications 1 à 7 pour distribuer de la matière pulvérisée pour inhalation.

14. Inhalateur selon l'une quelconque des revendications 8 à 13 apte à distribuer de la matière pulvérisée pour remise au système respiratoire supérieur.

15. Inhalateur selon la revendication 14 apte à délivrer des gouttelettes ayant un diamètre d'environ 10 à environ 25 micromètres au système respiratoire supérieur.

16. Dispositif selon l'une quelconque des revendications 1 à 7 apte à distribuer des parfums, arômes ou à permettre la délivrance inhalée d'un médicament.

17. Procédé de distribution de matière pulvérisée qui comprend : la fourniture de liquide à un site de pulvérisation et le fait de provoquer la pulvérisation du liquide en chargeant le site de pulvérisation à un potentiel électrique par induction en utilisant un moyen de charge espacé du site de pulvérisation, **caractérisé par** le fait de décharger électriquement partiellement ou intégralement de la matière pulvérisée pour empêcher un choc sur le moyen de charge d'induction.

18. Procédé selon la revendication 17 qui comprend le fait de fournir le liquide à pulvériser et d'induire le potentiel électrique au site de pulvérisation de façon à procurer des gouttelettes pour remise au système respiratoire supérieur.

19. Procédé selon la revendication 17 qui comprend la fourniture du liquide à pulvériser et l'induction du potentiel électrique au site de pulvérisation de façon à procurer des gouttelettes ayant un diamètre dans la gamme d'environ 10 à environ 25 micromètres pour délivrance au système respiratoire supérieur.
